# EUROPÄISCHE PATENTSCHRIFT

(11) **EP 0 708 072 B1**
(45) Veröffentlichungstag und Bekanntmachung des Hinweises auf die Patenterteilung: **22.04.1998**
(21) Anmeldenummer: 95112179.7
(22) Anmeldetag: 03.08.1995
(51) Int. Cl.: C07C 15/04, C07C 15/06, C07C 7/08, C10G 7/08

(54) **Verfahren zur Gewinnung von reinem Benzol und reinem Toluol**
Process for obtaining pure benzene and pure toluene
Procédé pour l'obtention de benzène pur et de toluène pur

(30) Priorität: 21.10.1994 DE 4437702
(43) Veröffentlichungstag der Anmeldung: 24.04.1996
(73) Patentinhaber: Krupp Uhde GmbH, 44141 Dortmund (DE)
(72) Erfinder: Leisse, Martin, D-45144 Essen (DE); Vollmer, Hans-Jürgen, D-45133 Essen (DE); Ranke, Uwe, D-45128 Essen (DE)
(74) Vertreter: Honke, Manfred, Dr.-Ing.

(56) Entgegenhaltungen:
- EP-A- 0 434 959
- WO-A-94/09878

## Beschreibung

Die Erfindung betrifft ein Verfahren zur Gewinnung von reinem Benzol und reinem Toluol aus einem aromatischen Kohlenwasserstoffe enthaltenden Vorprodukt, wobei das Vorprodukt in ein benzolreiches und ein toluolreiches Zwischenprodukt aufgetrennt wird, wobei das benzolreiche und das toluolreiche Zwischenprodukt einer Extraktivdestillationsverfahrensstufe zugeführt und getrennt verschiedenen Böden einer Extraktivdestillationskolonne aufgegeben werden, wobei eine Mischung aus reinem Benzol, reinem Toluol und Extraktionsmittel dem Sumpf der Extraktivdestillationskolonne entnommen wird und wobei das Extraktionsmittel in einer Stripperverfahrensstufe vom reinen Benzol und vom reinen Toluol abgetrennt und zur Extraktivdestillationsverfahrensstufe rückgeführt wird. - Reines Benzol und reines Toluol meint, daß das Benzol und Toluol rein im technischen Sinne ist, d.h., daß noch geringfügige Verunreinigungen mit Fremdsubstanzen vorliegen können. Als aromatische Kohlenwasserstoffe enthaltendes Vorprodukt wird in der Regel Kokereirohbenzol aus der Kohleverkokung und/oder Pyrolysebenzin aus der Erdölverarbeitung eingesetzt. Grundsätzlich sind aber auch beispielsweise Vergasungsbenzol aus der Vergasung von Kohle oder Erdöl oder katalytisches Reformat aus der Erdölverarbeitung einsetzbar. Diese Vorprodukte enthalten meist Restgasmengen aus der Herstellung der Vorprodukte. Solche Restgase sind beispielsweise Schwefelwasserstoff, Ammoniak und Wasserstoff. Man spricht dann von unstabilisierten Vorprodukten. Ein unstabilisiertes Vorprodukt muß jedoch vor der Weiterverarbeitung stabilisiert, d. h. von den Restgasen befreit werden, damit keine störenden Reaktionen bei der Weiterverarbeitung stattfinden können. Dies erfolgt in einer destillativen Stabilisierungsverfahrensstufe. Dabei wird das unstabilisierte Vorprodukt einer Stabilisierkolonne aufgegeben und das Restgas dem Kopf der Stabilisierkolonne entnommen und abgeführt. Das stabilisierte Vorprodukt fällt im Sumpf der Stabilisierkolonne an. In einer Extraktivdestillationsverfahrensstufe wird der Extraktivdestillationskolonne neben dem bzw. den aufzubereitenden Zwischenprodukten ein selektives Lösungsmittel mit polaren Gruppen als Extraktionsmittel aufgegeben, in der Regel am Kopf der Extraktionsdestillationskolonne. Dadurch werden die Partialdrucke von Nichtaromaten stärker erhöht als die Partialdrucke von Aromaten und so die diesbezügliche Trennung in der Extraktivdestillationskolonne gefördert. Die Nichtaromaten werden beim Kopf der Extraktivdestillationskolonne abgenommen und abgeführt. Das Extraktionsmittel mit darin gelösten Aromaten fällt im Sumpf der Extraktivdestillationskolonne an. Eine Stripperverfahrensstufe nutzt eine geeignet eingerichtete Destillationskolonne zur Abtrennung des Extraktionsmittels von den Aromaten. Das so erhaltene Gemisch aus Benzol und Toluol ist ohne weiteres praktisch vollständig in seine Komponenten auftrennbar.

Ein Verfahren der eingangs genannten Art ist aus der Praxis bekannt und im wesentlichen in der EP-A-434 959 A2 beschrieben. Gemäß dieser Literaturstelle wird die Auftrennung des stabilisierten Vorprodukts in ein benzolreiches und ein toluolreiches Zwischenprodukt mittels einer Destillationskolonne durchgeführt, wobei das benzolreiche Zwischenprodukt den Kopf der Destillationskolonne und das toluolreiche Zwischenprodukt einem Seitenabzug der Destillationskolonne entnommen werden, während hochsiedende Bestandteile des Vorproduktes im Sumpf der Destillationskolonne anfallen und abgeführt werden. In der Praxis ist bei dem insofern bekannten Verfahren eine destillative Stabilisierungsverfahrensgtufe der üblichen Art der Destillationskolonne vorgeschaltet. Dieses Verfahren hat sich grundsätzlich bewährt, ist jedoch hinsichtlich der Reinheit des erhaltenen Benzols und Toluols noch verbesserungsfähig, da allein der Anteil an nichtaromatischen Kohlenwasserstoffen im Benzol noch ca. 0,1 % und im Toluol noch ca. 0,2 % beträgt.

Aus der DE-A-15 43 119 ist ein Verfahren anderer Art zur Gewinnung von Benzol und Toluol aus einem aromatische Kohlenwasserstoffe enthaltenden Vorprodukt bekannt, welches sehr reines Benzol, jedoch unreines, mit ca. 1,5 Gew.-% nichtaromatischen Kohlenwasserstoffen befrachtetes Toluol liefert. Dieses Verfahren arbeitet mit einer ersten Destillationsverfahrensstufe, in welcher oberhalb von 150° C siedende Komponenten von den (stabilisierten) Vorprodukt abgetrennt werden. Das insofern vorgereinigte Vorprodukt wird ohne weitere Trennung einem einzigen Boden einer Extraktivdestillationskolonne aufgegeben. In der Praxis ist dieses Verfahren dadurch weiterentwickelt worden, daß die Extraktivdestillationsverfahrensstufe diskontinuierlich betrieben wird, wobei das beschriebene Verfahren von Zeit zu Zeit unterbrochen wird um das erhaltene unreine Toluol mittels der Extraktivdestillationsverfahrensstufe von den störenden nichtaromatischen Kohlenwasserstoffen zu befreien. Im Ergebnis erhält man sowohl sehr reines Benzol als auch sehr reines Toluol; nachteilig ist jedoch, daß aufgrund der diskontinuierlichen Verfahrensweise unter anderem große Tanks für die Zwischenlagerung von vorgereinigtem Vorprodukt bzw. unreinem Toluol erforderlich sind. Dies ist aufwendig. Zudem ist diese diskontinuierliche Verfahrensweise energetisch ungünstig, da viel Hochdruckdampf benötigt wird. Statt der diskontinuierlichen Arbeitsweise kann grundsätzlich auch eine zweite Extraktivdestillationsverfahrensstufe zur Nachbehandlung des unreinen Toluol eingerichtet sein. Dies ist jedoch sehr aufwendig.

Gegenüber dem eingangs genannten Stand der Technik liegt der Erfindung das technische Problem zugrunde, ein Verfahren zur Gewinnung von reinem Benzol und reinem Toluol aus einem aromatische Kohlenwasserstoffe enthaltenden Vorprodukt anzugeben, bei welchem das erhaltene Benzol und Toluol weniger Fremdstoffe, insbesondere weniger nichtaromatische Kohlenwasserstoffe, enthält, und zwar ohne wesentliche Erhöhung des Aufwandes.

Zur Lösung dieses technischen Problems lehrt die Erfindung, daß das Vorprodukt einer destillativen Stabilisierungsverfahrensstufe von Gasen befreit sowie in das benzolreiche und das toluolreiche Zwischenprodukt aufgetrennt wird, und daß einerseits das benzolreiche Zwischenprodukt direkt aus der destillativen Stabilisierungsverfahrensstufe der Extraktivdestillationsverfahrensstufe zugeführt wird und andererseits das toluolreiche Zwischenprodukt vor der Zufuhr zur Extraktivdestillationsverfahrensstufe in einer Vordestillationsverfahrensstufe von hochsiedenden Komponenten abgereichert wird. - Die Erfindung nutzt die Erkenntnis, daß eine ohnehin vorhandene destillative Stabilisierungsverfahrensstufe nicht nur zum Austreiben störenden Restgases, sondern auch zur gleichzeitigen Auftrennung des Vorproduktes in ein benzolreiches und toluolreiches Zwischenprodukt ausgebildet werden kann. Dadurch ist eine ansonsten zur Auftrennung des Vorproduktes eingesetzte Destillationsverfahrensstufe zur ausschließlichen Vorreinigung des toluolreichen Zwischenproduktes bezüglich hochsiedender Komponenten frei. Zudem läßt sich die Vordestillationsverfahrensstufe aufgrund des geringeren Mengenstroms kleiner dimensionieren. Es wird sehr reines Benzol und sehr reines Toluol erhalten bei kontinuierlicher Verfahrensweise, also ohne die Notwendigkeit aufwendiger Tanks zur Zwischenlagerung aber auch ohne die Notwendigkeit zusätzlicher Extraktivdestillationsverfahrensstufen. Im Ergebnis wird ein hinsichtlich der Reinheit verbessertes Endprodukt bei praktisch gleichbleibendem Aufwand erhalten. Auch der Energieaufwand bleibt gegenüber dem nächstliegenden Stand der Technik im wesentlichen gleich. Insgesamt wird durch die erfindungsgemäße Verfahrensführung ein überraschender synergistischer Effekt erhalten, da üblicherweise eine Verbesserung der Reinheit des Endprodukts, insbesondere bei ohnehin hohem Reinheitsniveau nur mit erheblicher Erhöhung des Aufwandes erreichbar ist.

Weiterbildungen des erfindungsgemäßen Verfahrens sind in den Patentansprüchen 2 bis 7 angegeben. Vorzugsweise wird als Extraktionsmittel N-Formylmorpholin verwendet. Gegenstand der Erfindung ist aber auch eine Vorrichtung zur Durchführung des Verfahrens, die dadurch gekennzeichnet ist, daß eine Stabilisierkolonne mit Seitenabzug zur Befreiung des Vorprodukts von Gasen und zur Auftrennung des Vorprodukts in ein benzolreiches sowie ein toluolreiches Vorprodukt eingerichtet ist, daß an den Sumpf der Stabilisierkolonne eine Vordestillationskolonne zur Befreiung des toluolreichen Zwischenproduktes von hochsiedenden Komponenten angeschlossen ist, daß eine Extraktivdestillationskolonne über die Zwischenproduktzuführanschlüsse einerseits an einen Seitenabzug der Stabilisierkolonne und andererseits an den Kopf der Vordestillationskolonne angeschlossen ist, daß an den Sumpf der Extraktivdestillationskolonne eine Stripperkolonne angeschlossen ist, wobei dem Sumpf der Stripperkolonne das Extraktionsmittel entnehmbar und über eine Extraktionsmittelzuführleitung der Extraktivdestillationskolonne zuführbar ist und wobei dem Kopf der Stripperkolonne eine Mischung aus einem Benzol und reinen Toluol entnehmbar ist. Weiterbildungen der erfindungsgemäRen Vorrichtung sind in den Patentansprüchen 9 und 10 angegeben.

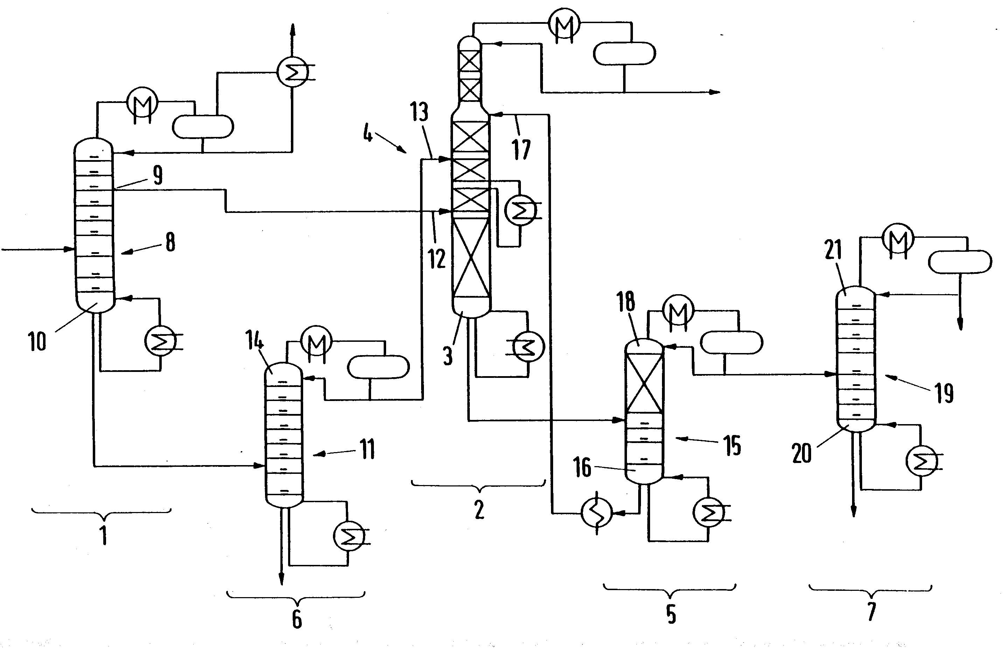

Im folgenden werden die erfindungsgemäße Vorrichtung sowie das mit ihr durchführbare erfindungsgemäße Verfahren anhand der lediglich ein Ausführungsbeispiel zeigenden Figur näher erläutert. Es zeigt die einzige Figur schematisch den Aufbau einer erfindungsgemäßen Vorrichtung.

Der Figur entnimmt man zunächst, daß grundsätzlich eine destillative Stabilisierungsverfahrensstufe 1, eine Vordestillationsverfahrensstufe 6, eine Extraktivdestillationsverfahrensstufe 2, eine Stripperverfahrensstufe 5 und eine Trennverfahrensstufe 7 eingerichtet sind. Die destillative Stabilisierungsverfahrensstufe 1 weist im wesentlichen eine Stabilisierkolonne 8 mit Seitenabzug 9 auf. Die Stabilisierkolonne ist zur Befreiung des Vorproduktes von Gasen und zur Auftrennung des Vorproduktes in ein benzolreiches sowie ein toluolreiches Zwischenprodukt eingerichtet. Sie weist ca. 60 praktische Böden auf, wobei ein Seitenabzug 9 bei ca. dem 52. praktischen Boden angeordnet ist. Die Vordestillationsverfahrensstufe 6 weist eine Vordestillationskolonne 11 zur Befreiung des toluolreichen Zwischenproduktes von hochsiedenden Komponenten auf. Die Vordestillationskolonne 11 ist mit ca. 65 praktischen Böden ausgestattet. Sie ist an den Sumpf 10 der Stabilisierkolonne 8 angeschlossen. Im Rahmen der Extraktivdestillationsverfahrensstufe 2 ist eine Extraktivdestillationskolonne 4 eingerichtet. Die Extraktivdestillationskolonne 4 ist mit zwei Zwischenproduktzuführanschlüssen 12, 13 einerseits an dem Seitenabzug 9 der Stabilisierkolonne 8 und andererseits an den Kopf 14 der Vordestillationskolonne 11 angeschlossen. Im einzelnen weist sie ca. 70 theoretische Böden auf, wobei der Zwischenproduktzuführanschluß 13 für das toluolreiche Zwischenprodukt ca. 20 theoretische Böden unterhalb der Extraktionsmittelzuführleitung 17 angeschlossen ist und wobei der Zwischenproduktzuführanschluß 12 für das benzolreiche Zwischenprodukt ca. 40 theoretische Böden unterhalb der Extraktionsmittelzuführleitung 17 angeordnet ist. Zwischen den Zwischenproduktzuführanschlüssen 12, 13 kann auf übliche Weise ein Kaminboden eingerichtet sein. Die Stripperverfahrensstufe 5 ist mit einer Stripperkolonne 15 ausgestattet, welche an den Sumpf 3 der Extraktivdestillationskolonne 4 angeschlossen ist. Dem Sumpf 16 der Stripperkolonne 15 ist das Extraktionsmittel entnehmbar und der Extraktionsmittelzuführleitung 17 der Extraktivdestillationskolonne 4 zuführbar. Dem Kopf 18 der Stripperkolonne 15 ist eine Mischung aus reinem Benzol und reinem Toluol entnehmbar. Die Trennverfahrensstufe 7 wird im wesentlichen durch die Fraktionierungskolonne 19 mit ca. 60 praktischen Böden gebildet. Die Fraktionierungskolonne 19 ist an den Kopf 18 der Stripperkolonne 15 angeschlossen. Dem Sumpf 20 der Fraktionierungskolonne 19 ist reines Toluol und dem Kopf 21 der Fraktionierungskolonne 19 reines Benzol entnehmbar.

In der Figur sind ohne Bezugszeichen mehrere Wärmetausch er sowie Kolonnenrückflüsse eingezeichnet. Diese sind ohne weiteres vom Durchschnittsfachmann einricht- und dimensionierbar. Es versteht sich, daß die Wärmeenergie optimal genutzt wird.

Mit der dargestellten erfindungsgemäßen Vorrichtung wird das erfindungsgemäße Verfahren im einzelnen wie folgend erläutert durchgeführt. Das Vorprodukt wird in der destillativen Stabilisierungsverfahrensstufe 1 von Gasen befreit. Dabei ist die destillative Stabilisierungsverfahrensstufe 1 zur Auftrennung des Vorprodukts in das benzolreiche und das toluolreiche Zwischenprodukt eingerichtet. Dies ist im einzelnen dadurch ausgebildet, daß das benzolreiche Zwischenprodukt über den Seitenabzug 9 der Stabilisierkolonne 8 entnommen wird und daß das toluolreiche Zwischenprodukt dem Sumpf 10 der Stabilisierkolonne 8 entnommen wird. Dabei wird die destillative Stabilisierverfahrensstufe 1 mit der Maßgabe betrieben, daß das toluolreiche Zwischenprodukt 10 bis 40 % Benzol, vorzugsweise ca. 20 % (± 5 %) Benzol, enthält. Dies ist gleichbedeutend mit der Maßgabe, daß im Vorprodukt enthaltenes Methylcyclohexan zu ca. 70 % in das toluolreiche Zwischenprodukt übergeht. Das benzolreiche Zwichenprodukt wird direkt aus der destillativen Stabilisierungsverfahrensstufe 1 der Extraktivdestillationsverfahrensstufe 2 zugeführt. Das toluolreiche Zwischenprodukt wird vor der Zufuhr zur Extraktivdestillationsverfahrensstufe 2 in der Vordestillationsverfahrensstufe 6 von hochsiedenden Komponenten abgereichert. Dabei ist die Vordestillationsverfahrensstufe 6 so eingerichtet und betrieben, daß Komponenten mit einem Siedepunkt von mehr als ca. 120° C von dem toluolreichen Zwischenprodukt abgetrennt werden. Solche Komponenten sind im wesentlichen aromatische Kohlenwasserstoffe mit mehr als 7 C-Atomen sowie höhere, nichtaromatische Kohlenwasserstoffe. In der Extraktivdestillationsverfahrensstufe 2 wird mit N-Formylmorpholin als Extraktionsmittel gearbeitet. Alternativ ist jedoch auch ein anderer Stoff aus der Gruppe "N-substituiertes Morpholin, dessen Substituenten weniger als 8 C-Atome aufweisen" oder Mischungen daraus verwendbar. Dieses Extraktionsmittel ist an sich und für sich bekannt, beispielsweise aus der DE-C-15 68 940. Das Extraktionsmittel wird beim Kopf der Extraktivdestillationskolonne 3 aufgegeben. Eine Mischung aus reinem Benzol, reinem Toluol und Extraktionsmittel wird dem Sumpf 3 der Extraktivdestillationskolonne 4 entnommen. In der Stripperverfahrensstufe 5 wird das Extraktionsmittel vom reinen Benzol und vom reinen Toluol abgetrennt und zur Extraktivdestillationsverfahrensstufe 2 rückgeführt. Die der Stripperverfahrensstufe 5 entnommene Mischung aus reinem Benzol und reinem Toluol wird in der Trennverfahrensstufe 7 in ihre Komponenten, d. h. reines Benzol und reines Toluol aufgetrennt. Dies gelingt ohne weiteres mit sehr hoher Selektivität.

Vergleichsversuche mit dem erfindungsgemäßen Verfahren einerseits und dem Verfahren gemäß der EP-A-434 959 andererseits ergaben unter anderem die in der folgenden Tabelle dargestellten Ergebnisse:

**Tabelle 1**

| | **erfindungsgemäßes Verfahren** | **Verfahren gemäß EP 0 434 959 A2** |
|---|---|---|
| Ausbeute Benzol | 98,90 % | 98,48 |
| Ausbeute Toluol | 94,85 % | 98,33 |
| NA in Benzol | < 0,012 % | 0,093 % |
| NA in Toluol | < 0,051 % | 0,202 % |

NA steht dabei für nichtaromatische Kohlenwasserstoffe. Bezüglich des Benzols erkennt man, daß nicht nur die Reinheit des Benzols erheblich verbessert ist, sondern daß überraschenderweise gleichzeitig eine Erhöhung der Ausbeute feststellbar ist. Dies ist insbesondere auch deshalb bedeutsam, da Benzolverluste aus Umweltschutzgründen aufwendig aus Abgasen entfernt werden müssen. Dieser Aufwand reduziert sich bei Erhöhung der Ausbeute und folglich Verringerung der Verluste. Zudem handelt es sich bei Benzol um den im Vergleich zum Toluol "wertvolleren" Stoff. Bezüglich des Toluols ist feststellbar, daß eine beträchtliche Erhöhung der Reinheit erreicht wird. Dies geht jedoch einher mit einer geringfügigen Verringerung Ausbeute. Ein Vergleich beider Verfahren hinsichtlich des apparativen und energetischen Aufwandes zeigt keine erheblichen Unterschiede.

Ein Vergleich des erfindungsgemäßen Verfahrens mit dem eingangs beschriebenen diskontinuierlichen Verfahren auf der Grundlage der Literaturstelle DE 15 43 119 A1 zeigt, daß zwar die erreichbaren Reinheiten des Benzols bzw. Toluols vergleichbar sind, daß der apparative Aufwand beim erfindungsgemäßen Verfahren jedoch erheblich geringer ist, da keine großen Tanks zur Zwischenlagerung benötigt werden. Zudem wird beim erfindungsgemäßen Verfahren ca. 1/3 weniger an wertvollen Hochdruckdampf benötigt.

## Patentansprüche

1. Verfahren zur Gewinnung von reinem Benzol und reinem Toluol aus einem aromatischen Kohlenwasserstoffe enthaltenden Vorprodukt,
wobei das Vorprodukt in ein benzolreiches und toluolreiches Zwischenprodukt aufgetrennt wird,
wobei das benzolreiche und das toluolreiche Zwischenprodukt einer Extraktivdestillationsverfahrensstufe (2) zugeführt und getrennt verschiedenen Böden einer Extraktivdestillationskolonne (4) aufgegeben werden,
wobei eine Mischung aus reinem Benzol, reinem Toluol und Extraktionsmittel dem Sumpf (3) der Extraktivdestillationskolonne (4) entnommen wird und
wobei das Extraktionsmittel in einer Stripperverfahrensstufe (5) von reinem Benzol und von reinem Toluol abgetrennt und zur Extraktivdestillationsverfahrensstufe (2) rückgeführt wird,
**dadurch gekennzeichnet**,
daß das Vorprodukt in einer destillativen Stabilisierungsverfahrensstufe (1) von Gasen befreit sowie in das benzolreiche und das toluolreiche Zwischenprodukt aufgetrennt wird und
daß einerseits das benzolreiche Zwischenprodukt direkt aus der destillativen Stabilisierungsverfahrensstufe (1) der Extraktivdestillationsverfahrensstufe (2) zugeführt wird und andererseits das toluolreiche Zwischenprodukt vor der Zufuhr zur Extraktivdestillationsverfahrensstufe (2) in einer Vordestillationsverfahrensstufe (6) von hochsiedenden Komponenten abgereichert wird.

2. Verfahren nach Anspruch 1, dadurch gekennzeichnet, daß das benzolreiche Zwischenprodukt einer Stabilisierkolonne (8) der destillativen Stabilisierverfahrensstufe (1) über einen Seitenabzug (9) entnommen wird, und daß das toluolreiche Zwischenprodukt dem Sumpf (10) der Stabilisierkolonne (8) entnommen wird.

3. Verfahren nach Anspruch 1 oder 2, dadurch gekennzeichnet, daß die destillative Stabilisierverfahrensstufe (1) mit der Maßgabe betrieben wird, daß das toluolreiche Zwischenprodukt 10 bis 40% Benzol, vorzugsweise ca. 20% Benzol, enthält.

4. Verfahren nach einem der Ansprüche 1 bis 3, dadurch gekennzeichnet, daß die Vordestillationsverfahrensstufe (6) mit der Maßgabe betrieben wird, daß Komponenten mit einem Siedepunkt von mehr als 120 °C von dem toluolreichen Zwischenprodukt abgetrennt werden.

5. Verfahren nach einem der Ansprüche 1 bis 4, dadurch gekennzeichnet, daß als Extraktionsmittel ein Stoff aus der Gruppe "N-substituiertes Morpholin, dessen Substituenten weniger als 8 C-Atome aufweisen" oder Mischungen daraus, verwendet wird.

6. Verfahren nach einem der Ansprüche 1 bis 5, dadurch gekennzeichnet, daß eine Extraktivdestillationskolonne (4) mit 70 theoretischen Böden verwendet wird, daß das toluolreiche Zwischenprodukt 20 theoretische Böden unterhalb des Extraktionsmittels aufgegeben wird, und daß das benzolreiche Zwischenprodukt 40 theoretische Böden unterhalb des Extraktionsmittels aufgegeben wird.

7. Verfahren nach einem der Ansprüche 1 bis 6, dadurch gekennzeichnet, daß die der Stripperverfahrensstufe (5) entnommene Mischung aus reinem Benzol und reinem Toluol in einer Trennverfahrensstufe (7) in ihre Komponenten aufgetrennt wird.

8. Vorrichtung zur Durchführung des Verfahrens nach einem der Ansprüche 1 bis 7, dadurch gekennzeichnet, daß eine Stabilisierkolonne (8) mit Seitenabzug (9) zur Befreiung des Vorprodukts von Gasen und zur Auftrennung des Vorprodukts in ein benzolreiches sowie ein toluolreiches Zwischenprodukt eingerichtet ist,
daß an den Sumpf (10) der Stabilisierkolonne (8) eine Vordestillationskolonne (11) zur Befreiung des toluolreichen Zwischenproduktes von hochsiedenden Komponenten angeschlossen ist,
daß eine Extraktivdestillationskolonne (4) über die Zwischenproduktzuführanschlüsse (12, 13) einerseits an den Seitenabzug (9) der Stabilisierkolonne (8) und andererseits an den Kopf (14) der Vordestillationskolonne (11) angeschlossen ist, und
daß an den Sumpf (3) der Extraktivdestillationskolonne (4) einer Stripperkolonne (15) angeschlossen ist, wobei dem Sumpf (16) der Stripperkolonne (15) das Extraktionsmittel entnehmbar und über eine Extraktionsmittelzuführleitung (17) der Extraktivdestillationskolonne (4) zuführbar ist und wobei den Kopf (18) der Stripperkolonne (15) eine Mischung aus reinem Benzol und reinem Toluol entnehmbar ist.

9. Vorrichtung nach Anspruch 8, dadurch gekennzeichnet, daß die Stabilisierkolonne (8) 60 praktische Böden aufweist, wobei der Seitenabzug (9) bei dem 52. praktischen Boden angeordnet ist,
daß die Vordestillationskolonne (11) 65 praktische Böden aufweist,
daß die Extraktivdestillationskolonne (4) 70 theoretische Böden aufweist, wobei der Zwischenproduktzuführanschluß (13) für das toluolreiche Zwischenprodukt 20 theoretische Böden unterhalb der Extraktionsmittelzuführleitung (17) angeordnet ist und wobei der Zwischenproduktzuführanschluß (12) für das toluolreiche Zwischenprodukt 40 theoretische Böden unterhalb der Extraktionsmittelzuführleitung (17) angeordnet ist.

10. Vorrichtung nach Anspruch 8 oder 9, dadurch gekennzeichnet, daß an den Kopf (18) der Stripperkolonne (15) eine Fraktionierungskolonne (19) mit 60 praktischen Böden angeschlossen ist, wobei dem Sumpf (20) der Fraktionierungskolonne (19) reines Toluol und dem Kopf (21) der Fraktionierungskolonne (19) reines Benzol entnehmbar sind.

## Claims

1. A process for producing pure benzene and pure toluene from a preliminary product containing aromatic hydrocarbons,
wherein the preliminary product is separated into a benzene-rich and into a toluene-rich intermediate,
wherein the benzene-rich and the toluene-rich intermediates are fed to an extractive distillation process stage (2) and are fed in separately to different plates of an extractive distillation column (4),
wherein a mixture of pure benzene, pure toluene and extractant is withdrawn from the bottom (3) of the extractive distillation column (4), and
wherein the extractant is separated from pure benzene and pure toluene in a stripping process stage (5) and is recycled to the extractive distillation process stage (2),
characterised in that
the preliminary product is freed from gases and is also separated into the benzene-rich and the toluene-rich intermediates in a distillative stabilisation process stage (1), and
that on the one hand the benzene-rich intermediate is fed directly from the distillative stabilisation process stage (1) to the extractive distillation process stage (2) and on the other hand the toluene-rich intermediate is stripped of high-boiling components in a pre-distillation process stage (6) before being fed to the extractive distillation process stage (2).

2. A process according to claim 1, characterised in that the benzene-rich intermediate is withdrawn from a stabilising column (8) of the distillative process stage (1) via a side take-off (9), and that the toluene-rich intermediate is withdrawn from the bottom (10) of the stabilising column (8).

3. A process according to claim 1 or 2, characterised in that the distillative stabilisation process stage (1) is operated with the proviso that the toluene-rich intermediate contains 10 to 40 % benzene, preferably about 20 % benzene.

4. A process according to any one of claims 1 to 3, characterised in that the pre-distillation process stage (6) is operated with the proviso that components with a boiling point higher than 120°C are separated from the toluene-rich intermediate.

5. A process according to any one of claims 1 to 4, characterised in that a substance from the group comprising N-substituted morpholine, the substituents of which comprise less than 8 C atoms, or mixtures thereof, is used as the extractant.

6. A process according to any one of claims 1 to 5, characterised in that an extractive distillation column (4) comprising 70 theoretical plates is used, that the toluene-rich intermediate is fed in 20 theoretical plates below the extractant, and that the benzene-rich intermediate is fed in 40 theoretical plates below the extractant.

7. A process according to any one of claims 1 to 6, characterised in that the mixture of pure benzene and pure toluene removed from the stripping process stage (5) is separated into its components in a separation process stage (7).

8. An apparatus for carrying out the process according to any one of claims 1 to 7, characterised in that a stabilising column (8) with a side take-off (9) is installed for freeing the preliminary products from gases and for separating the preliminary product into a benzene-rich and into a toluene-rich intermediate,
that a pre-distillation column (11) for freeing the toluene-rich intermediate from high-boiling components is connected to the bottom (10) of the stabilising column (8),
that an extractive distillation column (4) is connected, via the intermediate feed connections (12, 13), firstly to the side take-off (9) of the stabilising column (8) and secondly to the top (14) of the pre-distillation column (11), and
that a stripping column (15) is connected to the bottom (3) of the extractive distillation column (4), wherein the extractant can be withdrawn from the bottom (16) of the stripping column (15) and can be fed via an extractant feed line (17) to the extractive distillation column (4), and wherein a mixture of pure benzene and pure toluene can be withdrawn from the top (18) of the stripping column (15).

9. An apparatus according to claim 8, characterised in that the stabilising column (8) comprises 60 practical plates, wherein the side take-off (9) is situated at the 52nd practical plate,
that the pre-distillation column (11) comprises 65 practical plates,
that the extractive distillation column (4) comprises 70 theoretical plates, wherein the intermediate feed connection (13) for the toluene-rich intermediate is situated 20 theoretical plates below the extractant feed line (17), and wherein the intermediate feed connection (12) for the toluene-rich intermediate is situated 40 theoretical plates below the extractant feed line (17).

10. An apparatus according to claim 8 or 9, characterised in that a fractionating column (19) comprising 60 practical plates is connected to the top (18) of the stripping column (15), wherein pure toluene can be withdrawn from the bottom (20) of the fractionating column (19) and pure benzene can be withdrawn from the top (21) of the fractionating column (19).

## Revendications

1. Procédé d'extraction de benzène pur et de toluène pur a partir d'une première fraction contenant des hydrocarbures aromatiques, la première fraction étant séparée en un produit intermédiaire riche en benzène et un produit intermédiaire riche en toluène, le produit intermédiaire riche en benzène et le produit intermédiaire riche en toluène étant soumis à une étape de procédé de distillation extractive (2) et chargés séparément sur différents plateaux d'une colonne de distillation extractive (4), un mélange de benzène pur, de toluène pur et de solvant d'extraction étant soutiré au fond (3) de la colonne de distillation extractive (4), et le solvant d'extraction étant séparé du benzène pur et du toluène pur dans une étape de procédé de stripage (5) et ramené à l'étape de procédé de distillation extractive (2), **caractérisé en ce** que la première fraction est libérée des gaz dans une étape de procédé de stabilisation distillative (1) et séparée en produits intermédiaires riches en benzène et en toluène, que d'une part le produit intermédiaire riche en benzène est amené directement de l'étape de procédé de stabilisation distillative (1) à l'étape de procédé de distillation extractive (2) et que d'autre part le produit intermédiaire riche en toluène est appauvri des constituants lourds avant l'amenée à l'étape de procédé de distillation extractive (2) dans une étape de procédé de première distillation (6).

2. Procédé selon la revendication 1, caractérisé en ce que le produit intermédiaire riche en benzène est prélevé dans une colonne de stabilisation (8) de l'étape de procédé de stabilisation distillative (1) par l'intermédiaire d'un soutirage latéral (9), et que le produit intermédiaire riche en toluène est soutiré au fond (10) de la colonne de stabilisation (8).

3. Procédé selon la revendication 1 ou 2, caractérisé en ce que l'étape de procédé de stabilisation distillative (1) est mise en oeuvre de manière que le produit intermédiaire riche en toluène contient 10 à 40 % de benzène, de préférence environ 20 % de benzène.

4. Procédé selon l'une des revendications 1 à 3, caractérisé en ce que l'étape de procédé de première distillation (6) est mise en oeuvre de manière que les constituants ayant un point d'ébullition de plus de 120°C sont séparés du produit intermédiaire riche en toluène.

5. Procédé selon l'une des revendications 1 à 4, caractérisé en ce qu'il utilise comme solvant d'extraction un composé choisi dans le groupe des 〈〈 morpholines N-substitués dont les substituants comportent moins de 8 atomes de C 〉〉 ou un mélange de celles-ci.

6. Procédé selon l'une des revendications 1 à 5, caractérisé en ce qu'il utilise une colonne de distillation extractive (4) avec 70 plateaux théoriques, que le produit intermédiaire riche en toluène est chargé 20 plateaux théoriques en dessous du solvant d'extraction, et que le produit intermédiaire riche en benzène est chargé 40 plateaux théoriques en dessous du solvant d'extraction.

7. Procédé selon l'une des revendications 1 à 6, caractérisé en ce que le mélange de benzène pur et de toluène pur soutiré dans l'étape de procédé de stripage (5) est séparé en ses constituants dans une étape de procédé de séparation (7).

8. Dispositif pour la mise en oeuvre du procédé selon l'une des revendications 1 à 7, caractérisé en ce qu'une colonne de stabilisation (8) avec soutirage latéral (9) est agencée pour libérer la première fraction des gaz et pour séparer la première fraction en un produit intermédiaire riche en benzène et un produit intermédiaire riche en toluène, qu'au fond (10) de la colonne de stabilisation (8) est raccordée une colonne de première distillation (11) pour libérer le produit intermédiaire riche en toluène des constituants lourds, qu'une colonne de distillation extractive (4) est raccordée, par l'intermédiaire des raccords d'amenée de produit intermédiaire (12, 13), au soutirage latéral (9) de la colonne de stabilisation (8) d'une part et à la tête (14) de la colonne de première distillation (11) d'autre part, et qu'au fond (3) de la colonne de distillation extractive (4) est raccordée une colonne de stripage (15), le solvant d'extraction pouvant être soutiré au fond (16) de la colonne de stripage (15) et amené à la colonne de distillation extractive (4) par l'intermédiaire d'une conduite d'amenée de solvant d'extraction (17), et un mélange de benzène pur et de toluène pur pouvant être soutiré à la tête (18) de la colonne de stripage (15).

9. Dispositif selon la revendication 8, caractérisé en ce que la colonne de stabilisation (8) présente 60 plateaux pratiques, le soutirage latéral (9) étant disposé au 52ème plateau pratique, que la colonne de première distillation (11) présente 65 plateaux pratiques, que la colonne de distillation extractive (4) présente 70 plateaux théoriques, le raccord d'amenée de produit intermédiaire (13) pour le produit intermédiaire riche en toluène étant disposé 20 plateaux théoriques en dessous de la conduite d'amenée de solvant d'extraction (17), et le raccord d'amenée de produit intermédiaire (12) pour le produit intermédiaire riche en toluène étant placé 40 plateaux théoriques en dessous de la conduite d'amenée de solvant d'extraction (17).

10. Dispositif selon la revendication 8 ou 9, caractérisé en ce qu'à la tête (18) de la colonne de stripage (15) est raccordée une colonne de fractionnement (19) avec 60 plateaux pratiques, du toluène pur pouvant être soutiré au fond (20) de la colonne de fractionnement (19) et du benzène pur pouvant être soutiré en tête (21) de la colonne de fractionnement (19).
